# EUROPEAN PATENT SPECIFICATION

(11) **EP 4 185 694 B1**
(45) Date of publication and mention of the grant of the patent: **18.02.2026**
(21) Application number: 21751524.6
(22) Date of filing: 23.07.2021
(51) Int. Cl.: C12N 15/113, A61K 31/712, A61P 9/00

(54) **COMBINATORIAL INHIBITION OF MIRNAS FOR TREATMENT OF HEART FAILURE**
KOMBINATORISCHE HEMMUNG VON MIRNAS ZUR BEHANDLUNG VON HERZVERSAGEN
INHIBITION COMBINATOIRE DE MIARN POUR LE TRAITEMENT DE L'INSUFFISANCE CARDIAQUE

(30) Priority: 23.07.2020 EP 20187308; 21.01.2021 EP 21152755
(43) Date of publication of application: 31.05.2023
(73) Proprietor: Johann Wolfgang Goethe-Universität Frankfurt, 60323 Frankfurt am Main (DE)
(72) Inventor: KRISHNAN, Jaya, 61476 Kronberg im Taunus (DE); YUAN, Ting, 65428 Rüsselsheim (DE); DIMMELER, Stefanie, 60323 Frankfurt am Main (DE)
(74) Representative: Schiweck Weinzierl Koch Patentanwälte Partnerschaft mbB
(86) International application number: PCT/EP2021/070705
(87) International publication number: WO 2022/018269

(56) References cited:
- WO-A1-2010/144485
- WO-A1-2013/192486
- WO-A1-2014/201301
- WO-A1-2018/017483
- WO-A2-2009/062169
- WO-A2-2010/126370
- WO-A2-2013/192576
- BAOFENG YANG ET AL: "The muscle-specific microRNA miR-1 regulates cardiac arrhythmogenic potential by targeting GJA1 and KCNJ2", NATURE MEDICINE, vol. 13, no. 4, 1 April 2007 (2007-04-01), pages 486 - 491, XP055032924, ISSN: 1078-8956, DOI: 10.1038/nm1569
- ZHANG YING ET AL: "Metformin Protects against H2O2-Induced Cardiomyocyte Injury by Inhibiting the miR-1a-3p/GRP94 Pathway", MOLECULAR THERAPY, vol. 13, 6 September 2018 (2018-09-06), US, pages 189 - 197, XP055813465, ISSN: 2162-2531, DOI: 10.1016/j.omtn.2018.09.001
- LUO SHUILIAN ET AL: "Rescuing infusion of miRNA-1 prevents cardiac remodeling in a heart-selective miRNA deficient mouse", BIOCHEMICAL AND BIOPHYSICAL RESEARCH COMMUNICATIONS, vol. 495, no. 1, 5 November 2017 (2017-11-05), Amsterdam NL, pages 607 - 613, XP055812669, ISSN: 0006-291X, DOI: 10.1016/j.bbrc.2017.11.029
- LIU QIONG ET AL: "Soluble epoxide hydrolase inhibitors might prevent ischemic arrhythmias via microRNA-1 repression in primary neonatal mouse ventricular myocytes", vol. 13, no. 3, 6 January 2017 (2017-01-06), GB, pages 556 - 564, XP055813458, ISSN: 1742-206X, Retrieved from the Internet <URL:https://pubs.rsc.org/en/content/articlepdf/2017/mb/c6mb00824k> DOI: 10.1039/C6MB00824K
- HONG TING ET AL: "A Novel Anti-Coagulative Nanocomplex in Delivering miRNA-1 Inhibitor Against Microvascular Obstruction of Myocardial Infarction", ADVANCED HEALTHCARE MATERIALS, vol. 9, no. 11, 27 April 2020 (2020-04-27), DE, pages 1901783, XP055813651, ISSN: 2192-2640, DOI: 10.1002/adhm.201901783

## Description

The present invention relates to a combination of inhibitory nucleic acid agents directed at a combination of miRNAs for treatment of heart failure. The combination of inhibited miRNAs comprises one of the groups selected from miR-1a and miR-15b, or miR-1a, miR-15b and miR-27b. The combinatorial inhibition of these miRNAs is shown to increase cardiomyocyte proliferation.

### Background of the Invention

Cardiovascular diseases are the leading causes of death in the world. Particularly heart failure is the major cause of cardiovascular morbidity and mortality. Heart failure results from the maladaptive remodeling of the heart that caused by myocardial infarction, chronic hypertension, cardiac fibrosis and inflammation. Loss of cardiomyocytes is a main hallmark of heart failure. However, there is a limited cardiomyocytes proliferation capacity in adult heart as well as in failing human heart. Therefore, regenerative approaches to replenish the lost cardiomyocytes would be necessary to recover the contractile capacity of the heart. Thus, targeting cardiomyocytes proliferation is one of the potential new therapeutic strategies for myocardial regeneration and repair.

Small regulatory microRNAs (miRNAs) are small, about 22 nucleotides, non-coding RNAs that suppress gene expression, which enhance the degradation or decay of their target protein-coding mRNAs. miRNAs regulate gene expression at the post-transcriptional level by binding to 3'-untranslated regions of target mRNAs. Many miRNAs have been shown that were regulated in cardiomyopathy, and microRNA overexpression or inhibition of single miRNAs can interfere with the development of heart failure by targeting key genes involved in inflammation, metabolism and cardiac function. Given their central role in regulation of gene expression in health and disease, miRNAs have emerged as potential therapeutic targets for a huge variety of disease. WO 2013 192 576 A2 lists several antisense oligonucleotides of miRNAs in treatment of cardiovascular diseases. WO 2009 062 169 A8 shows inhibition of the miR-15 family may be applicable in treatment of heart disease. WO 2010 144 485 A1 provides formulations of miRNA inhibitors with improved stability and potency.

Based on the above-mentioned state of the art, the objective of the present invention is to provide means and methods to treat heart failure. This objective is attained by the subject-matter of the independent claims of the present specification.

### Summary of the Invention

A first aspect of the invention relates to a combination of nucleic acid agents for use in treatment or prevention of heart disease. Each of the nucleic acid agents is directed at and capable of specifically inhibiting and/or degrading a target microRNA. The combination of nucleic acid agents is capable of inhibiting and/or degrading a combination of microRNAs within a cell. The combination of inhibited or degraded microRNAs is
a. miR-1a, and
b. miR-15b, or
miR-15b and miR-27b.

A second aspect of the invention relates to an expression construct, or a plurality of expression constructs, encoding, under control of a promoter operable in a human myocardial cell, a combination of nucleic acid agents for use in treatment or prevention of heart disease. Each of the nucleic acid agents is directed at and capable of specifically inhibiting and/or degrading a target microRNA, thereby inhibiting and/or degrading a combination of microRNAs. The combination of microRNAs comprises
a. miR-1a, and
b. miR-15b, or
miR-15b and miR-27b.

A third aspect of the invention relates to a pharmaceutical composition for use in treatment or prevention of heart disease comprising the combination of nucleic acid agents according to the first aspect, or the plurality of expression constructs according to the second aspect.

In another embodiment, the present invention relates a pharmaceutical composition comprising at least one of the compounds of the present invention or a pharmaceutically acceptable salt thereof and at least one pharmaceutically acceptable carrier, diluent or excipient.

### Detailed Description

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The terms *capable of forming a hybrid* or *hybridizing sequence* in the context of the present specification relate to sequences that under the conditions existing within the cytosol of a mammalian cell, are able to bind selectively to their target sequence. Such hybridizing sequences may be contiguously reverse-complimentary to the target sequence, or may comprise gaps, mismatches or additional non-matching nucleotides. The minimal length for a sequence to be capable of forming a hybrid depends on its composition, with C or G nucleotides contributing more to the energy of binding than A or T/U nucleotides, and on the backbone chemistry.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

The term *miRNA* (microRNA) in the context of the present specification relates to a small non-coding RNA molecule (containing about 22 nucleotides) that functions in RNA silencing and post-transcriptional regulation of gene expression.

The term *antisense oligonucleotide* in the context of the present specification relates to an oligonucleotide having a sequence substantially complimentary to, and capable of hybridizing to, an RNA. Antisense action on such RNA will lead to modulation, particular inhibition or suppression of the RNA's biological effect. If the RNA is an mRNA, expression of the resulting gene product is inhibited or suppressed. Antisense oligonucleotides can consist of DNA, RNA, nucleotide analogues and/or mixtures thereof. The skilled person is aware of a variety of commercial and non-commercial sources for computation of a theoretically optimal antisense sequence to a given target. Optimization can be performed both in terms of nucleobase sequence and in terms of backbone (ribo, deoxyribo, analogue) composition. Many sources exist for delivery of the actual physical oligonucleotide, which generally is synthesized by solid state synthesis. Examples of antisense oligonucleotides of the present invention are antisense oligomers made of DNA, DNA having phosphorothioate modified linkages in their backbone, ribonucleotide oligomers, RNA comprising bridged or locked nucleotides, particularly wherein the ribose ring is connected by a methylene bridge between the 2'-O and 4'-C atoms, RNA having phosphorothioate modified linkages in their backbone or any mixture of deoxyribonucleotide and ribonucleotide bases as an oligomer.

In certain embodiments, the antisense oligonucleotide of the invention comprises analogues of nucleic acids such as phosphotioates, 2'O-methylphosphothioates, peptide nucleic acids (PNA; N-(2-aminoethyl) -glycine units linked by peptide linkage, with the nucleobase attached to the alpha-carbon of the glycine) or locked nucleic acids (LNA; 2'O, 4'C methylene bridged RNA building blocks). The antisense sequence may be composed partially of any of the above analogues of nucleic acids, with the rest of the nucleotides being "native" ribonucleotides occurring in nature, or may be mixtures of different analogues, or may be entirely composed of one kind of analogue.

The term *nucleic acid expression vector* in the context of the present specification relates to a plasmid or a viral genome, which is used to transfect (in case of a plasmid) or transduce (in case of a viral genome) a target cell with a certain gene of interest. The gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

Sequences similar or homologous (e.g., at least about 70% sequence identity) to the sequences disclosed herein are also part of the invention. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

In the context of the present specification, the term *hybridizing sequence* encompasses a polynucleotide sequence comprising or essentially consisting of RNA (ribonucleotides), DNA (deoxyribonucleotides), phosphothioate deoxyribonucleotides, 2'-O-methyl-modified phosphothioate ribonucleotides, LNA and/or PNA nucleotide analogues.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.
miR-1a:
   hsa-mir-1-3p: Accession ID: MIMAT0000416; UGGAAUGUAAAGAAGUAUGUAU (SEQ ID NO 019).
   mmu-mir-1-3p: Accession ID: MIMAT0000123; UGGAAUGUAAAGAAGUAUGUAU (SEQ ID NO 020).
miR-15b:
   hsa-miR-15b-5p: Accession ID: MIMAT0000417; UAGCAGCACAUCAUGGUUUACA (SEQ ID NO 021).
   mmu-miR-15b-5p: Accession ID: MIMAT0000124; UAGCAGCACAUCAUGGUUUACA (SEQ ID NO 022).
miR-27b:
   hsa-miR-27b-5p: Accession ID: MIMAT0004588; AGAGCUUAGCUGAUUGGUGAAC (SEQ ID NO 023).
   mmu-miR-27b-5p: Accession ID: MIMAT0004522; AGAGCUUAGCUGAUUGGUGAAC (SEQ ID NO 024).
miR-34a:
   hsa-miR-34a-5p: Accession ID: MIMAT0000255; UGGCAGUGUCUUAGCUGGUUGU (SEQ ID NO 025).
   mmu-miR-34a-5p: Accession ID: MIMAT0000542; UGGCAGUGUCUUAGCUGGUUGU (SEQ ID NO 026).

A first aspect of the invention relates to a combination of nucleic acid agents for use in treatment or prevention of heart disease. Each of the nucleic acid agents is directed at and capable of specifically inhibiting and/or degrading a target microRNA. The combination of nucleic acid agents is capable of inhibiting and/or degrading a combination of microRNAs within a cell. The combination of inhibited or degraded microRNAs is
a. miR-1a, and
b. miR-15b, or
miR-15b and miR-27b.

In certain embodiments, the combination of microRNAs comprises miR-1a and miR-15b.

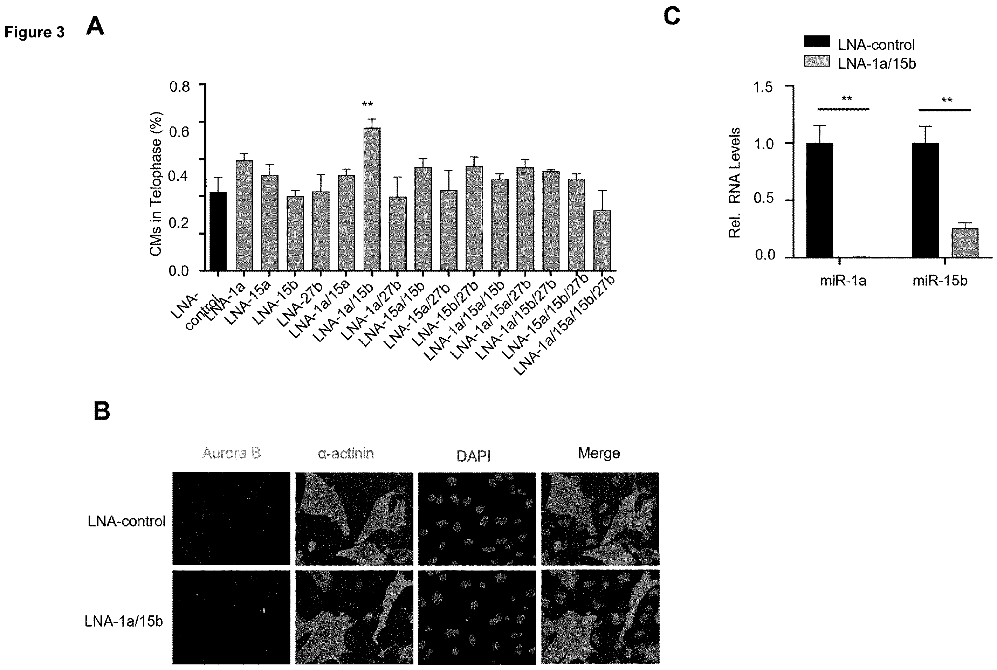

The combinations of nucleic acid agents were tested in P7 rat cardiomyocytes, when cell division had ceased (Figure 3). This situation resembles the treatment situation in heart failure best, because also in the heart failure treatment situation, cell division of cardiomyocytes has ceased. The inventors found that the combination of miR-1a and miR-15b induces the highest cell division rate (Figure 3), measured as percentage of cardiomyocytes in Telophase.

In certain embodiments, the nucleic acid agents are antisense nucleotides.

In certain embodiments, each nucleic acid agent comprises a hybridizing sequence of nucleotides. In certain embodiments, each nucleic acid agent essentially consists of the hybridizing sequence of nucleotides. The hybridizing sequence of nucleotides is capable of forming a hybrid with its respective target microRNA.

In certain embodiments, each nucleic acid agent comprises a (different) sequence selected from the sequences SEQ ID NO 001-004. In certain embodiments, each nucleic acid agent essentially consists of a (different) sequence selected from the sequences SEQ ID NO 001-004.

In certain embodiments, each nucleic acid agent comprises a (different) sequence selected from the sequences SEQ ID NO 001-002. In certain embodiments, each nucleic acid agent essentially consists of a (different) sequence selected from the sequences SEQ ID NO 001-002.

In certain embodiments, one of the nucleic acid agents comprises or essentially consists of, one or several locked nucleic acid (LNA) and/or peptide nucleic acid (PNA) moieties. In certain embodiments, all of said nucleic acid agents comprise or essentially consist of, one or several locked nucleic acid (LNA) and/or peptide nucleic acid (PNA) moieties. In certain embodiments, the nucleic acid agents essentially consist of locked nucleic acid (LNA) moieties.

In certain embodiments, one of the nucleic acid agents comprises one or several thiophosphate linkages connecting adjacent (deoxy)ribonucleoside moieties or ribonucleotide analogue moieties. In certain embodiments, several or all of the nucleic acid agents comprise one or several thiophosphate linkages connecting adjacent (deoxy)ribonucleoside moieties or ribonucleotide analogue moieties.

In certain embodiments, one of the nucleic acid agents comprises a LNA moiety connected to an adjacent ribonucleoside or ribonucleotide analogue moiety by a thiophosphate bond. In certain embodiments, one of the nucleic acid agents comprises 2, 3 or 4 LNA moieties connected by thiophosphate bonds on either end of the nucleic acid agent.

In certain embodiments, one of said nucleic acid agents is expressed intracellularly from an exogenous (transgene) expression construct comprising a sequence encoding a hybridizing sequence of nucleotides under control of a promoter operable in a human myocardial cell.

In certain embodiments, several or all of said nucleic acid agents are expressed intracellularly from an exogenous (transgene) expression construct comprising a sequence encoding a hybridizing sequence of nucleotides under control of a promoter operable in a human myocardial cell.

A second aspect of the invention relates to an expression construct, or a plurality of expression constructs, encoding, under control of a promoter operable in a human myocardial cell, a combination of nucleic acid agents for use in treatment or prevention of heart disease. Each of the nucleic acid agents is directed at and capable of specifically inhibiting and/or degrading a target microRNA, thereby inhibiting and/or degrading a combination of microRNAs. The combination of microRNAs comprises
a. miR-1a, and
b. miR-15b, or
miR-15b and miR-27b.

In certain embodiments, the combination of microRNAs comprises miR-1a and miR-15b.

In certain embodiments, each and all of the expression constructs are comprised within a single nucleic acid molecule.

In certain embodiments, the single nucleic acid molecule comprising all of the expression constructs is a polycistronic construct, which has several open-reading frames, one for each of the combination of nucleic acid agents of the invention.

In certain embodiments, the nucleic acid agents or the expression construct are bound to a nanoparticle or encapsulated by a particulate structure selected from a virus and a liposome.

A liposome is a spherical vesicle having at least one lipid bilayer. Liposomes can be prepared by disrupting biological membranes (e.g. by sonication). The major types of liposomes are the multilamellar vesicle (MLV, with several lamellar phase lipid bilayers), the small unilamellar liposome vesicle (SUV, with one lipid bilayer), the large unilamellar vesicle (LUV), and the cochleate vesicle.

A nanoparticle is a particle of matter that is between 1 and 100 nanometres (nm) in diameter. In certain embodiments, the nanoparticle is composed of a material selected from silver, gold, hydroxyapatite, clay, titanium dioxide, silicon dioxide, zirconium dioxide, carbon, diamond, aluminium oxide and ytterbium trifluoride.

In certain embodiments, the virus is selected from a retrovirus, a lentivirus, an adenovirus, and an adeno-associated virus, or a hybrid of the afore-mentioned. In certain embodiments, the virus selectively transduces cardiomyocytes.

In certain embodiments, the nanoparticle, or the particulate structure comprise a ligand facilitating delivery to or entry of the nanoparticle or particulate structure into a cardiomyocyte. In certain embodiments, the ligand is selected from an anti-CD71 Fab' fragment and an anti-cTnl Fab' fragment. These ligands function by targeting a cardiomyocyte-specific cell surface receptor for targeted delivery.

In certain embodiments, the promoter allows for heart-specific expression of said nucleic acid agents. In certain embodiments, the promoter is a heart-tissue-specific RNA Polymerase 2 promoter. In certain embodiments, the promoter is selected from Myosin Light Chain 2v (MLC2v) promoter, cardiac Troponin T (cTnT) promoter, Myosin Heavy Chain alpha (MHCa) promoter, and Titin (Ttn) promoter.

In certain embodiments, the heart disease is characterized by a loss of cardiomyocytes. In certain embodiments, the heart disease is heart failure characterized by a loss of cardiomyocytes. In certain embodiments, the heart failure is a result of myocardial infarction, stenosis, congenital disease, inflammation and/or sepsis.

In certain embodiments, the heart disease is characterized by hypoxia of cardiomyocytes.

Heart failure (HF) occurs when the heart is unable to pump sufficiently to maintain blood flow to meet the body's needs and leads to death if untreated. HF is the culmination of disease progression in indications including coronary artery disease, ischemic heart disease, aortic stenosis, hypertension and metabolic disease. Heart failure may be caused by a loss of cardiomyocytes. Cardiomyocytes are often lost due to insufficient supply with oxygen (hypoxia). The remaining cardiomyocytes are triggered to divide and replace the lost cardiomyocytes using the combination of nucleic acid agents of the present invention.

A third aspect of the invention relates to a pharmaceutical composition for use in treatment or prevention of heart disease comprising the combination of nucleic acid agents according to the first aspect, or the plurality of expression constructs according to the second aspect.

In certain embodiments, the pharmaceutical composition is administered to the heart.

### Medical treatment, Dosage Forms and Salts

Similarly, a dosage form for the prevention or treatment of heart disease is provided, comprising an antisense molecule according to any of the above aspects or embodiments of the invention.

The skilled person is aware that any specifically mentioned drug may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

### Pharmaceutical Compositions and Administration

Another aspect of the invention relates to a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant and easily handleable product.

The pharmaceutical composition can be formulated for oral administration, parenteral administration, or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

The invention is further illustrated by the following examples and figures, from which further embodiments and advantages can be drawn. These examples are meant to illustrate the invention but not to limit its scope.

### Description of the Figures

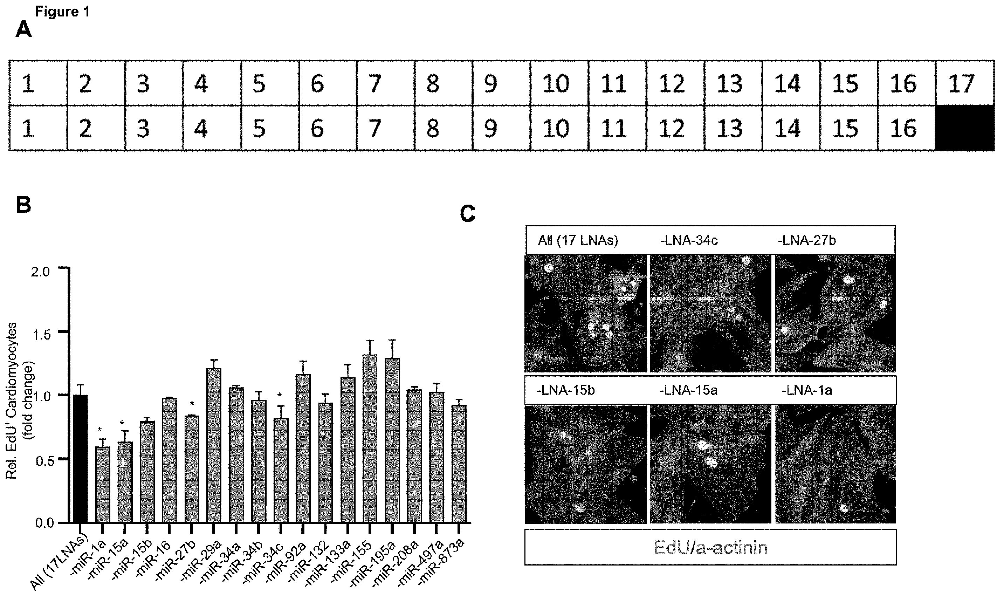

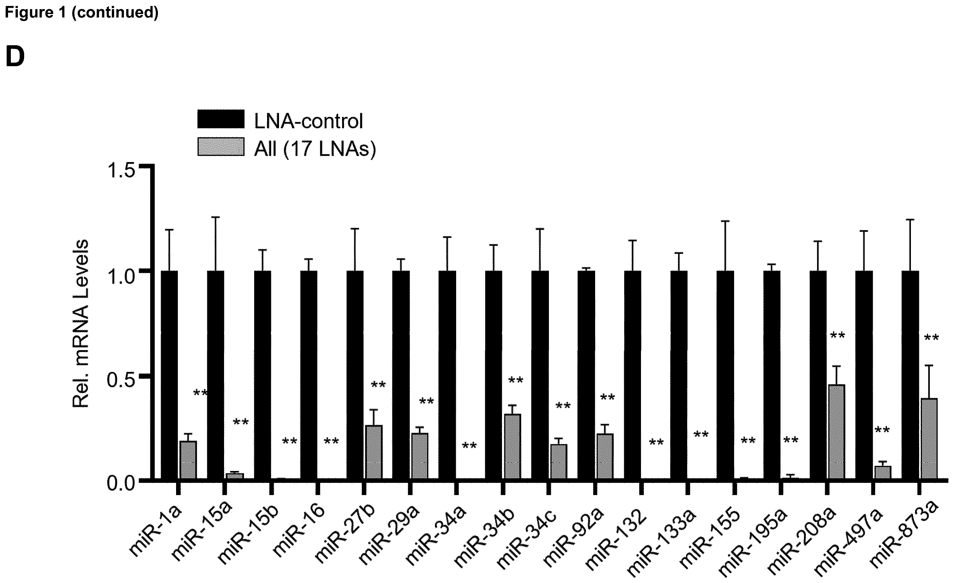

- Figure 1: Narrowing down the candidate miRNA LNAs by EdU screening. The inventors selected 17 miRNAs as candidates for factors that negatively regulate cardiomyocyte proliferation. To knockdown these miRNAs in rat cardiomyocytes, locked nucleic acids (LNAs) was used from Exiqon in the study. Rat neonatal cardiomyocytes were treated with removal of individual factors from the pool of 17 LNAs, while the cells were treated with 17 LNAs
- tar108wo: together as control. After 2 days treatment, the DNA synthesis (EdU) was analyzed by double staining for EdU (green) and α-actinin (red) (A, B, C). (D) The miRNA expression levels were analyzed by qRT-PCR. Data are presented as mean ± SEM. *P<0.05, **P<0.01. Statistical analysis was performed by the 2-tailed Student's t test.

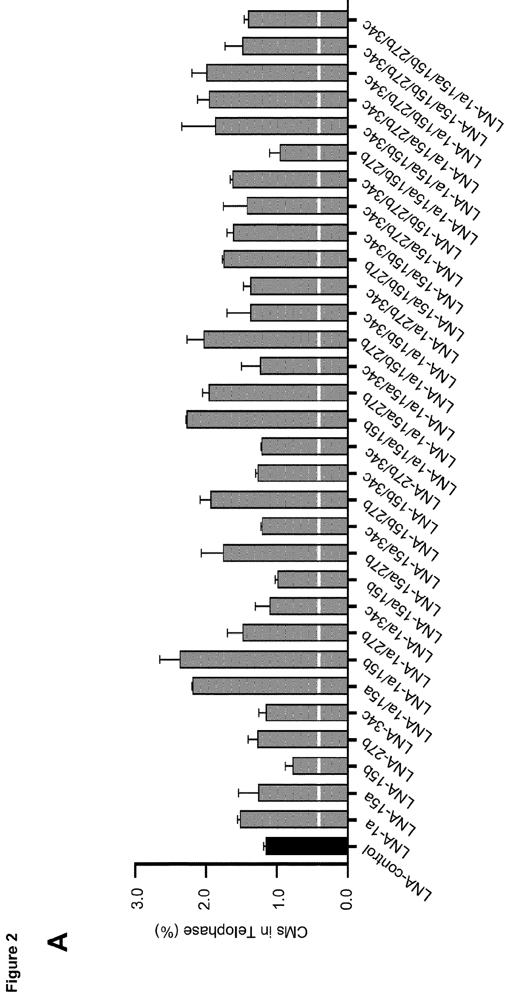

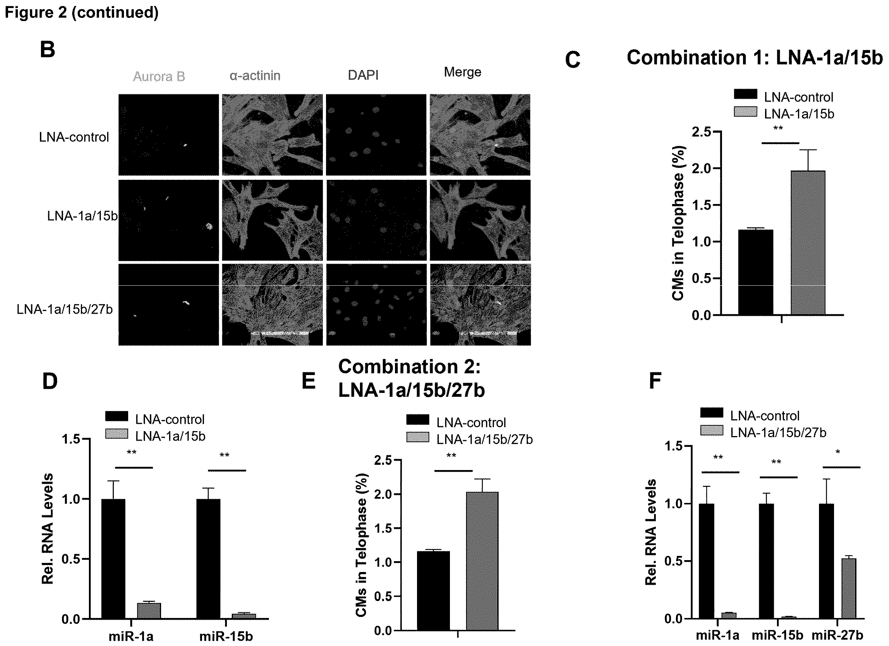

- Figure 2: Cell proliferation on rat cardiomyocytes *in vitro.* Based on EdU screening, the inventors selected 5 LNAs (LNA-1a, LNA-15a, LNA-15b, LNA-27b, LNA-34c) which are necessary for DNA synthesis. To identify the best combinations, all combinatorial LNAs were treated in the neonatal rat cardiomyocytes, and then the cardiomyocyte proliferation was analyzed by triple staining for Aurora B (green), α-actinin (red) and DAPI (blue) (A, B). Based on Aurora B screening, the inventors selected 2 combinations which could increase cardiomyocyte proliferation. The cardiomyocyte proliferation was analyzed by Aurora B (B, C, E), and miRNA expression levels were anlalyzed by qRT-PCR (D, F). *P<0.05, **P<0.01.
- Figure 3: Cell proliferation on 7-day-old rat cardiomyocytes *in vitro.* The inventors selected LNA-1a, LNA-15a, LNA-15b and LNA-27b, and test the effect of all combinations in 7-day-old rat cardiomyocytes. The rat cardiomyocytes were treated with all combinatorial LNAs for '2 days, and cardiomyocyte proliferation was analyzed by triple staining for Aurora B (green), α-actinin (red) and DAPI (blue) (A, B). miRNA expression levels were analyzed by qRT-PCR (C). *P<0.05, **P<0.01.

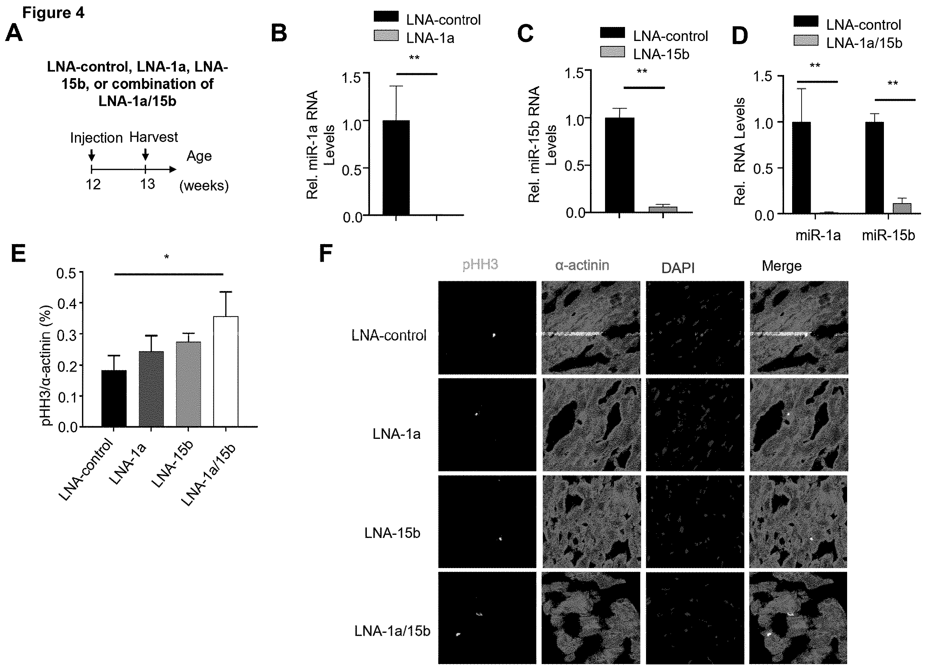

- Figure 4: miRNA expression levels *in vivo.* 12-week-old mice were injected with individual LNA-1a, LNA-15b, combination of LNA-1a and LNA-15b, or LNA-control. After 7 days injection, the left ventricles were harvested and the miRNA gene expression were analyzed by qRT-PCR (A, B, C and D). The cardiomyocyte proliferation was analyzed by triple staining for pHH3 (green), α-actinin (red) and DAPI (blue) (E and F). Data are expressed as mean ± SEM. *P<0.05, **P<0.01.

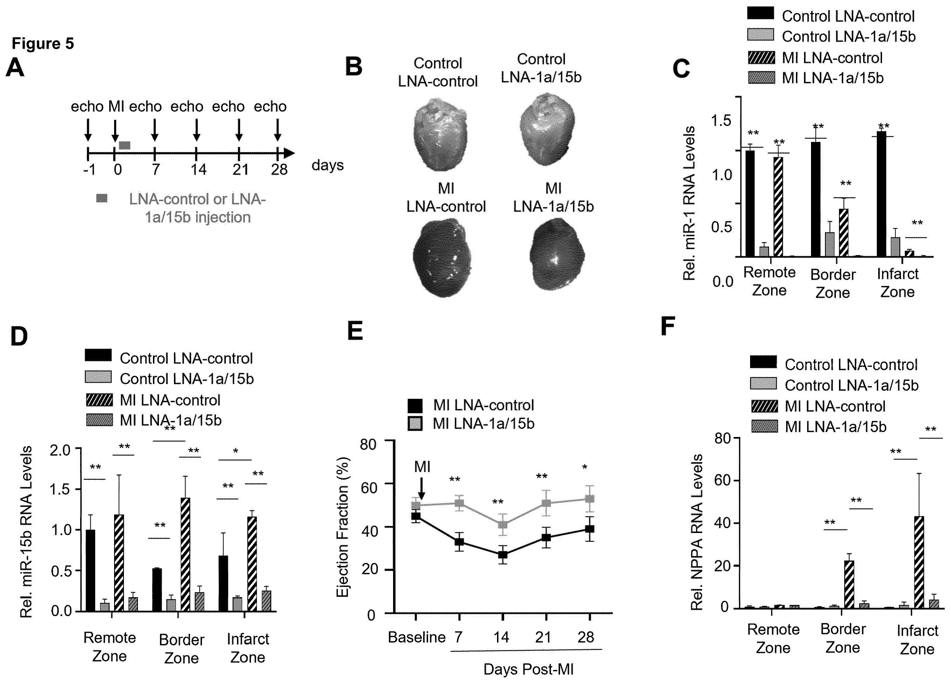

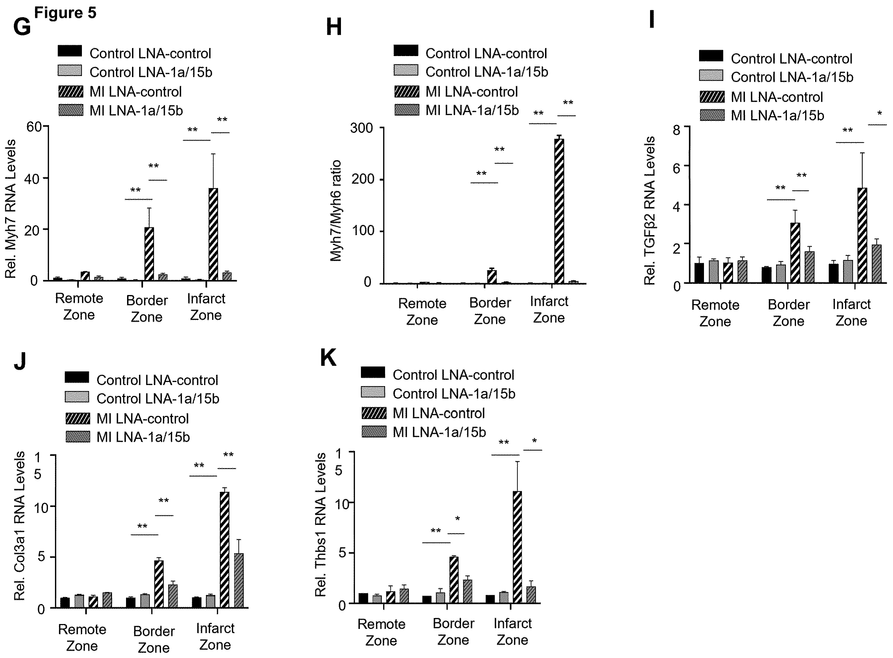

- Figure 5: 12-week-old mice were subjected to the Myocardial infarction, and then were injected with combination of LNA-1a and LNA-15b, or LNA-control. Echocardiography was performed at day 0, 7, 14, 21 and 28 (A). (B) After 28 days injection, whole hearts harvested from mice. (C, D) Analysis of miR-1a and miR-15b expression after MI injected with LNA-control or LNA-1a/15b in Remote Zone, Border Zone and Infarct Zone. (E) Longitudinal monitoring of cardiac % Ejection fraction (%EF). (F, G, H) Pathological remodeling marker genes (NPPA, my77, and Myh7/myh6 ratio) were quantified by qRT-PCR after LNAs injection and MI injury in Remote Zone, Border Zone and Infarct Zone. (I, J, K) Fibrotic remodeling marker genes (TGFβ2, Col3a1 and Thbs1) were quantified by qRT-PCR after LNAs injection and MI injury in Remote Zone, Border Zone and Infarct Zone. MI: Myocardial Infarction, NPPA: natriuretic peptide, myh7: beta-myosin heavy chain, myh6: beta-myosin heavy chain (Myh7)n=5 for sham and n=7 for MI. Data are expressed as means ± SEM. *p<0.05, **p<0.01. All by one-way ANOVA analyses followed by either a Dunnett's multiple comparison post-test.

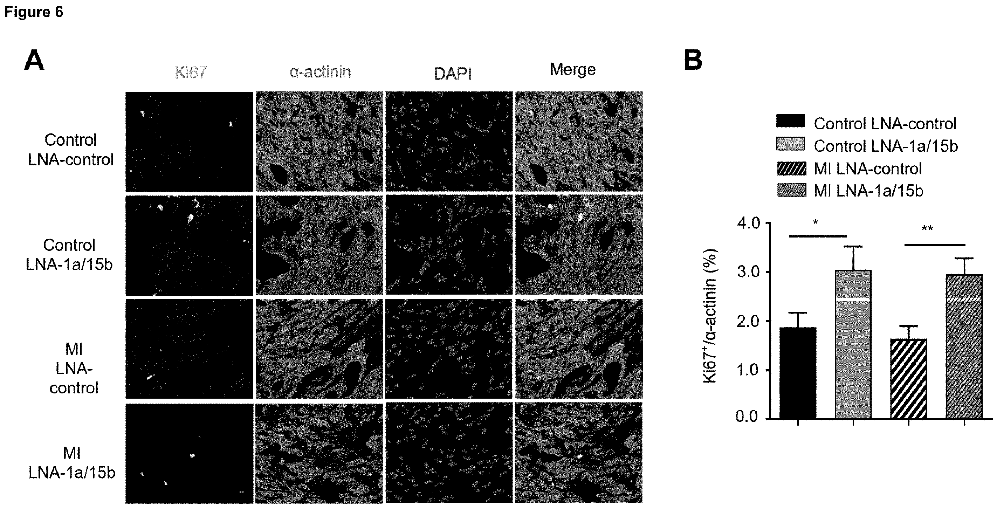

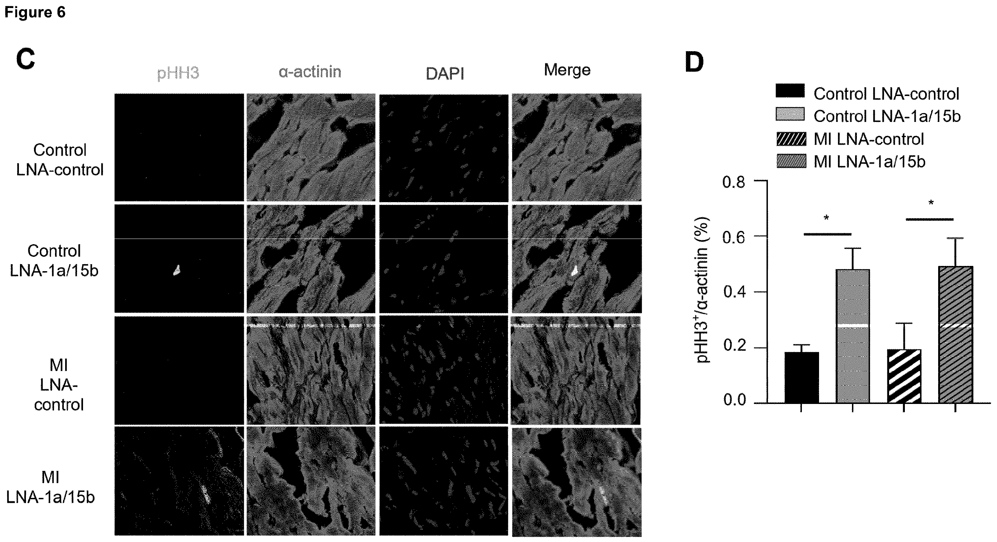

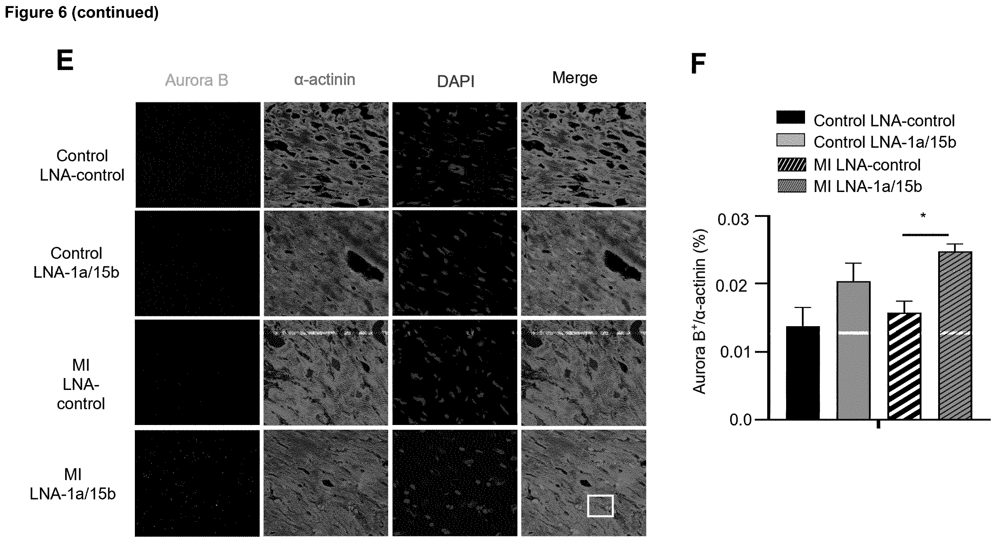

- Figure 6: Inhibition of miR-1a and miR-15b increases cardiomyocyte proliferation after myocardial infarction *in vivo.* (A) Representative immunofluorescence images of Ki67 on heart sections of adult hearts injected with LNA-1a/15b or control 28-days post MI injury. Ki67 labels proliferating cells (green); cardiomyocyte-specific α-actinin (red) and DAPI (Blue). (B) Quantification of percentages of Ki67⁺ cardiomyocytes. (C) Representative immunofluorescence images of pHH3 on heart sections of adult hearts injected with LNA-1a/15b or control LNA-control 28-days post MI injury. pHH3 labels proliferating cells (green); cardiomyocyte-specific α-actinin (red) and DAPI (Blue). (D) Quantification of percentages of pHH3⁺ cardiomyocytes. (E) Representative immunofluorescence images of Aurora B on heart sections of adult hearts. Aurora B labels proliferating cells (green); cardiomyocyte-specific α-actinin (red) and DAPI (Blue). (F) Quantification of percentages of Aurora B⁺ cardiomyocytes. Data are expressed as means ± SEM. *p<0.05, **p<0.01. All by one-way ANOVA analyses followed by either a Dunnett's multiple comparison post-test.

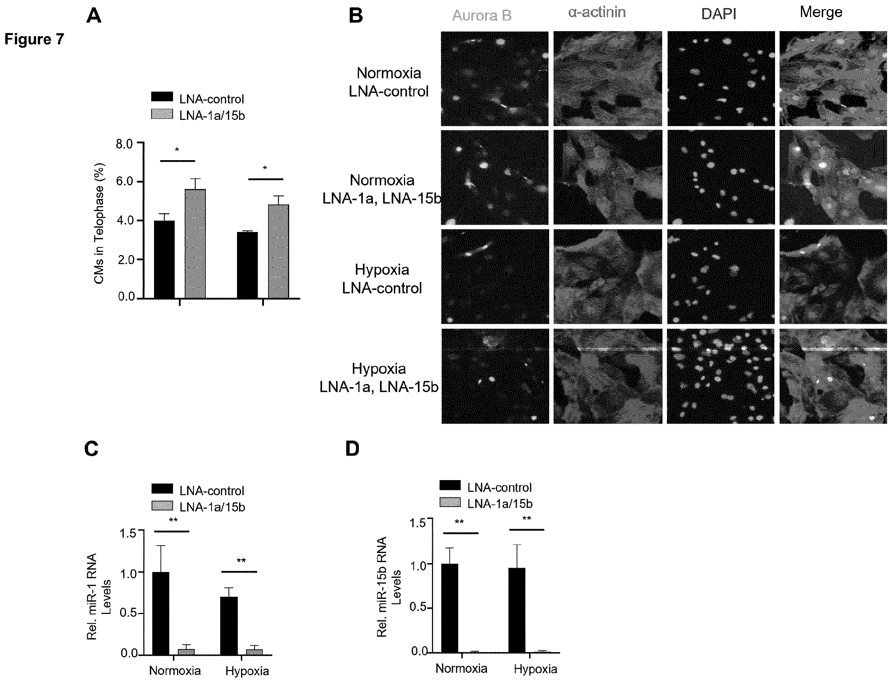

- Figure 7: Cell Proliferation on human iPS-CMs *in vitro.* Human iPSC-CMs were treated with either combination of LNA-1a and LNA-15b, or LNA-control, and then the cells were exposed to normoxia (20% O₂) or hypoxia (3% O₂) for 2 days. Cardiomyocytes proliferation was analyzed by Aurora B (A, B). The miRNA expression levels were analyzed by qRT-PCR (C, D). Data are expressed as mean ± SEM. *P<0.05, **P<0.01.

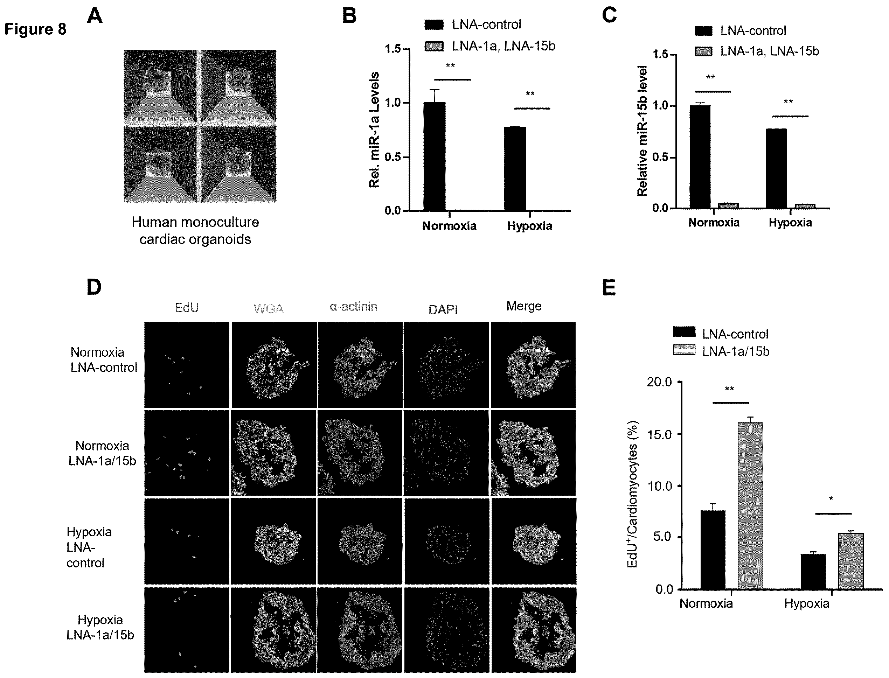

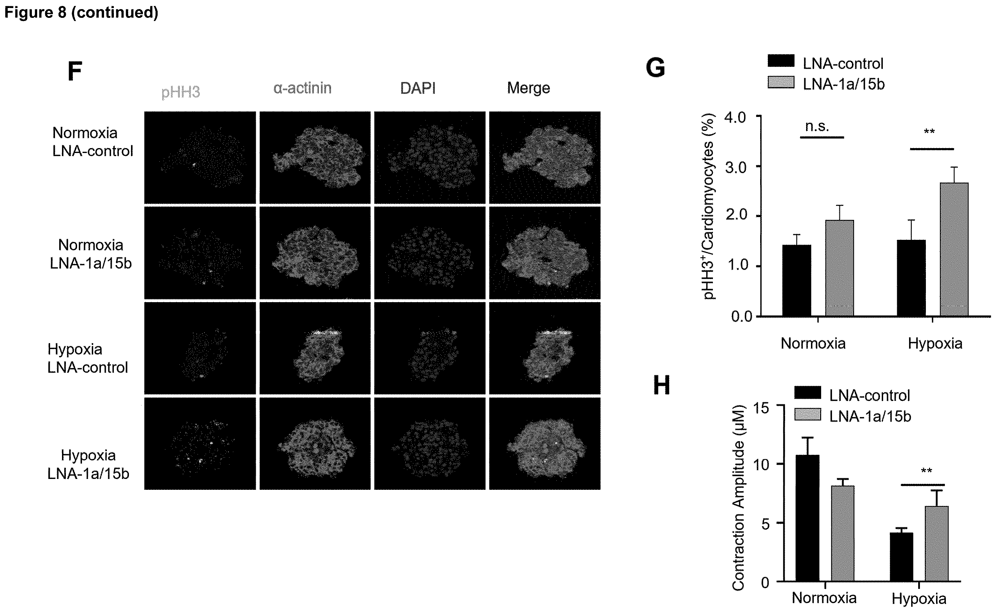

- Figure 8: Effects of combination of LNA-1a and LNA-15b on cardiomyocyte proliferation and cardiac function in human mimetic tissues. Human cardiac organoids (A) were treated with either LNA-1a/15b or LNA-control and then were cultured in normoxia and hypoxia for 3 days. (B, C) The miR-1a and miR-15b expression levels were analyzed by qRT-PCR. (D) Representative immunofluorescence images of EdU on sections of human cardiac organoids treated with LNA- 1a/15b or control LNA-control. Wheat-Germ-Agglutinin (WGA) marks cell surfaces (green); EdU labels proliferating cells (Magenta); cardiomyocyte-specific α-actinin (red), and DAPI (Blue). (E) Quantification of percentages of EdU⁺ cardiomyocytes. (F) Representative immunofluorescence images of pHH3 on sections of monoculture cardiac organoids treated with LNA-1a/15b or LNA-control in both normoxia and hypoxia. pHH3 labels proliferating cells (green); cardiomyocyte-specific α-actinin (red) and DAPI (Blue). (G) Quantification of of percentages of pHH3⁺ cardiomyocytes. (H) Human monoculture cardiac organoids were treated with LNA-1a/15b or LNA-control for 3 days in both normoxia and hypoxia, and then the contractility assays were performed by determining the amplitude peak of contracting monoculture cardiac organoids. Data are expressed as means ± SEM. *p<0.05, **p<0.01. All by one-way ANOVA analyses followed by either a Dunnett's multiple comparison post-test. hiPSC-CMs, human induced pluripotent stem cell-derived cardiomyocytes.

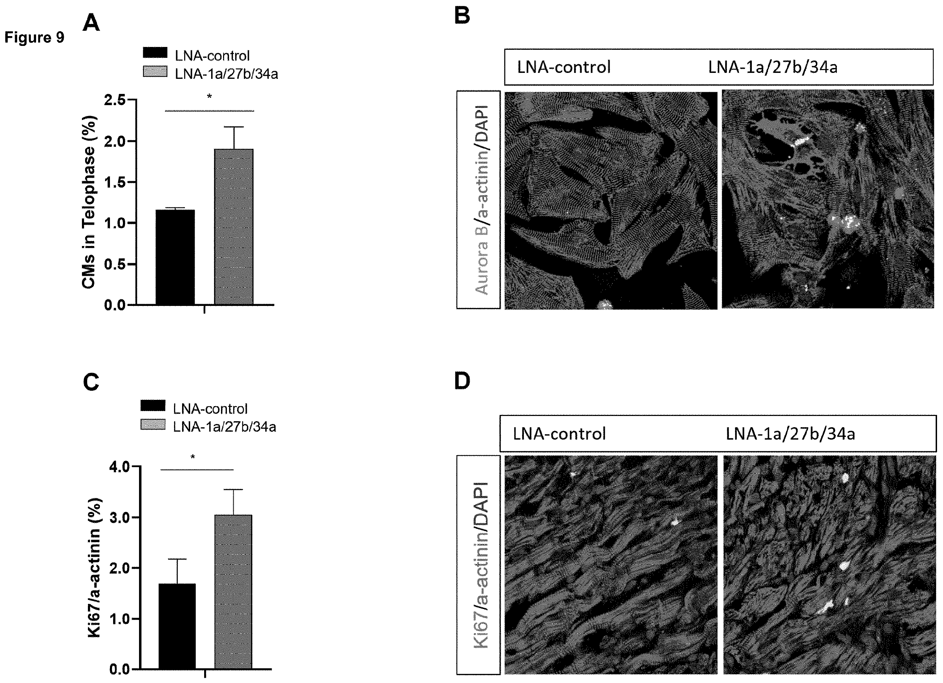

- Figure 9: Effects of combination of LNA-1a, LNA-27b and LNA-34a on cardiomyocyte proliferation *in vitro* and *in vivo.* Rat neonatal cardiomyocytes were treated with either combination of LNA-1a, LNA-27b and LNA-34a, or LNA-control for 2 days. Cardiomyocytes proliferation was analyzed by triple staining for Aurora B (green), α-actinin (red), and DAPI (blue) (A and B). 12-week-old mice were injected with either combination of LNA-1a, LNA-27b and LNA-34a, or LNA-control for 1 week. Cardiomyocytes proliferation was analyzed by triple staining for Ki67 (green), α-actinin (red), and DAPI (blue) (A and B). Data are expressed as mean ± SEM. *P<0.05, **P<0.01.

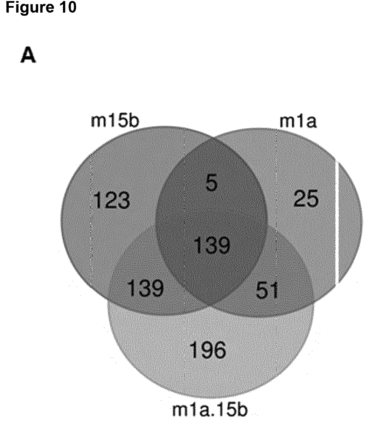

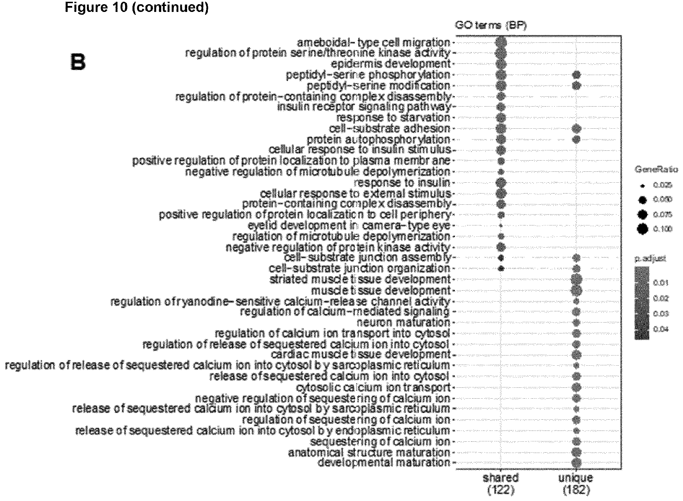

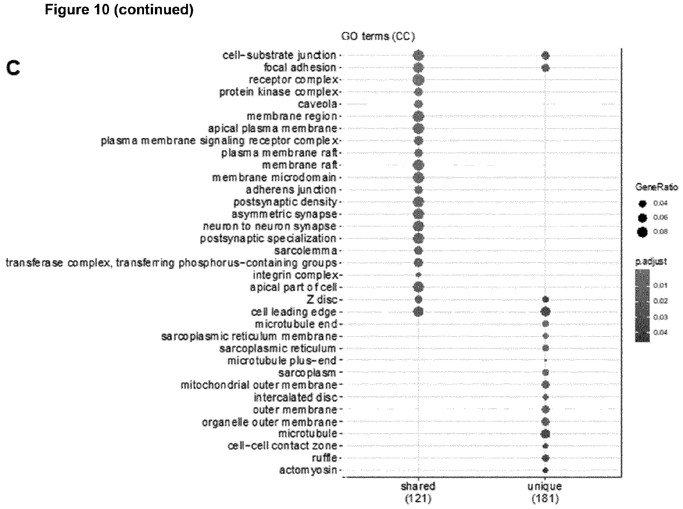

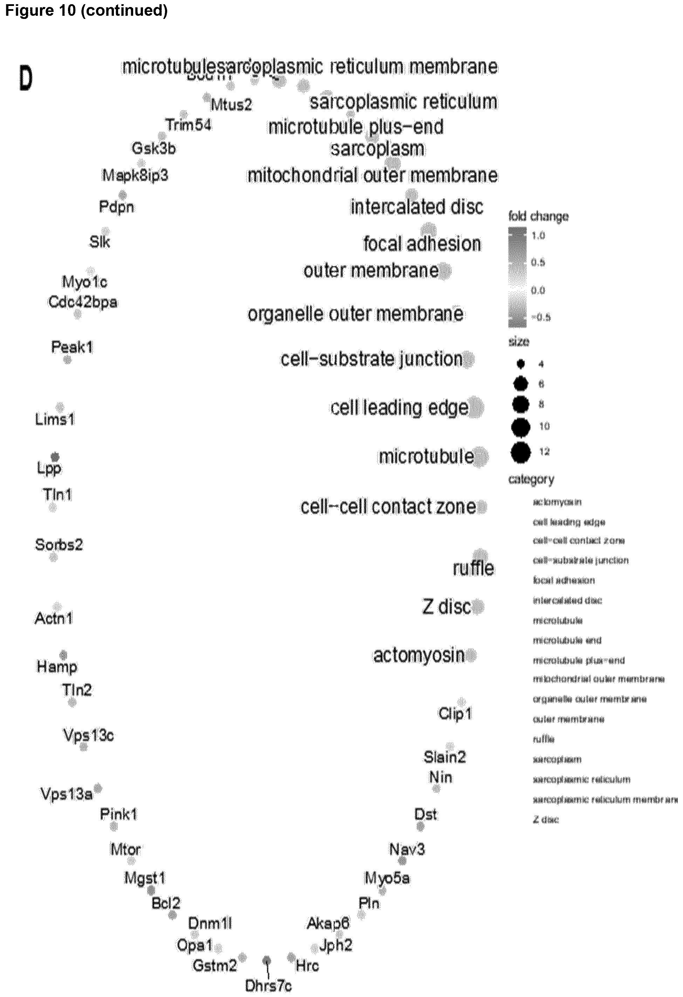

- Figure 10: A: Deep RNA sequencing (RNAseq) was performed on adult cardiomyocytes treated with miR1a-LNA or miR-15a-LNA alone, or in combination (miR1a/15b-LNA). Venn diagram (A) reveals unique and common genes under the respective conditions. B: Gene Ontology (GO) analysis of differentially expressed genes corresponding to Biological Processes (BP) pathways indicates the emergence and activation of unique pathways in cardiomyocytes treated with miR1a/15b (unique), compared to pathways common to miR1a-LNA, miR-15a-LNA and miR1a/15b-LNA treatment (shared) (B). C and D: GO analysis of Cellular Components (CC) present upon respective LNA treatment indicates emergence and activation of unique pathways in cardiomyocytes treated with miR1a/15b (unique), compared to pathways common to miR1a-LNA, miR-15a-LNA and miR1a/15b-LNA treatment (shared) (C). Genes unique to miR1a/15b-LNA treatment and their connectivity to specific Cellular Components (CC) pathways are depicted in (D).

### Examples

### Example 1: Screening for inhibition of miRNAs that promote cardiomyocyte proliferation

The inventors selected 17 miRNAs as candidates to induce cardiomyocyte proliferation. To evaluate these 17 candidate miRNAs, they were inhibited by locked nucleic acid (LNA)-based antimiR (LNA-miRNA) in rat cardiomyocytes *in vitro.* The inventors developed a combinatorial approach to detect proliferation in cardiomyocytes. To determine which of the 17 candidate LNAs were critical, the inventors treated the neonatal rat cardiomyocytes (NRCs, P1) with all 17 LNAs together as a control, and examined the cardiomyocyte proliferation of withdraw of individual LNA from the pool of 17 LNAs by using EdU (5-Ethynyl-2'-deoxyuridine) incorporation (Figure 1A, 1 B and 1C). The initial assessment for EdU was done within 48 h after LNAs treatment, and the rat cardiomyocytes were marked by sarcomeric α-actinin. Removal of individual LNA targeting miR-1a, miR-15a, miR-15b, miR-27b, or miR-34c resulted in decreased numbers of cardiomyocytes in S-phase, as shown in Figure 1B and 1C. Removal of the remaining LNAs did not significantly affect cardiomyocyte proliferation. The miRNA expression levels were detected by quantitative real time PCR (Figure 1D). These results indicate that miR-1a, miR-15a, miR-15b, miR-27b and miR-34c play important roles in the DNA synthesis in rat cardiomyocytes.

### Example 2: Combination inhibition of miR-1a and miR-15b induces cardiomyocyte proliferation

While EdU serves as an important maker for de novo DNA synthesis and incorporation in S-phase, it precludes the determination of cell-cycle completion and daughter cell formation. In order to ascertain that our miRNA drive cytokinesis and cell-cycle completion, the inventors utilized Aurora B as marker. Aurora B is physically associated with inner centromere protein and survivin in mitosis. It associates with centromeric heterochromatin early in mitosis, transfer to the central spindle, and finally localize to the contractile ring and midbody. Thus, Aurora B staining and localization at contractile ring and midbody serves as marker for cytokinesis and cell-cycle completion.

To determine which combination could enhance cytokinesis, 31 conditions corresponding to combinations of the 5 candidate LNAs targeting miR-1a, miR-15a, miR-15b, miR-27b and miR-34c were tested in 1-day-old rat cardiomyocytes. Cardiomyocyte proliferation was assessed by co-localization of contractile ring and midbody Aurora B staining with sarcomeric α-actinin to marking cells in cytokinesis (Figure 2A and 2B). These results showed that 2 combinations (LNA-1a and LNA-15b; LNA-1a, LNA-15b and LNA-27b) have higher numbers of cardiomyocytes in telophase than other combinations (Figure 2B, 2C and 2E). The miRNA expression levels were shown in Figure 2D and 2F.

Furthermore, all combinations from LNAs targeting miR-1a, miR-15a, miR-15b and miR-27b were screened in 7-day-old rat cardiomyocytes (P7), when cell division had ceased. After 48 h, the cardiomyocyte proliferation was assessed by Aurora B staining. Combination of LNA-1a and LNA-15b resulted in further enhancement in Aurora B in telophase in 7-day-old rat cardiomyocytes (Figure 3A and 3B). The miR-1a and miR-15b expression levels were shown in Figure 2D and 2F. These data suggest that the combination inhibition of miR-1a and miR-15b effectively and efficaciously enhances cardiomyocyte proliferation in both P1 and P7 rat cardiomyocytes *in vitro.*

### Example 3: Inhibition of miR-1a and miR-15b enhances cardiac function after myocardial infarction in vivo

To knockdown the miR-1a and miR-15b in vivo, LNA-based antimiRs (LNA-1a, LNA-15b) were administered by intraperatoneal injection (Figure 4A). Single and combinatorial LNAs were tested in the 12-week-old mice, and the heart were harvested 7 days after injection. Quantitative real-time PCR confirmed the successful delivery of either one or both LNAs compared to LNA-control (Figure 4B, 4C and 4D). To determine the effect of either one or both LNAs on cardiomyocyte proliferation, the inventors examined the expression of pHH3, and found increased pHH3 signal in mouse hearts after LNA-1a/15b injection compared to LNA-control (Figure 4E and 4F).

To determine the effect of miR-1a and miR-15b inhibition on cardiomyocyte proliferation and cardiac function after injury, miR-1a and miR-15b were inhibited by intraperitoneal injection of 4 hours after myocardial infarction (MI) in 12-week-old mice (Figure 5A), and the hearts were harvested at day 28 (Figure 5B). Echocardiography was performed at day 0, 7, 14, 21 and 28. After 28 days, the hearts were harvested and divided into three parts: Remote Zone, Border Zone and Infarct Zone. miRNA levels of miR-1a and miR-15b were significantly decreased in the Remote Zone, Border Zone and Infarct Zone in LNA-1a/15b treatment mice compared to the control group (Figure 5c and 5D).

Cardiac function was measured by echocardiography, and the inventors found that mice injected with LNA-1a/LNA-15b exhibited a dramatic increase in ejection fraction (EF), as measure of cardiac contractile function, compared to controls (Figure 5E). The inventors further examined the expression of molecular markers of pathologic hypertrophy, and we found that brain natriuretic peptide (NPPA) and beta-myosin heavy chain (Myh7) were reduced after LNA-1a/15b injection in both border zone and infarct zone compared to the control LNA injection (Figure 5F and 5G). The MI-induced Myh7:Myh6 ratio was significantly reduced by combinatiorial inhibition of miR-1a and miR-15b (Figure 5H). Furthermore, the expression levels of fibrosis-related genes (TGFβ2, Col3a1 and Thbs1) were repressed by LNA-1a/15b injection in both border zone and infarct zone after MI (Figure 5I, 5J and 5K). These results indicate that LNA-1a/15b combinatorial treatment produced a significantly greater protective effect in MI.

### Example 4: Inhibition of miR-1a and miR-15b increases cardiomyocyte proliferation after myocardial infarction in vivo

The inventors have demonstrated that combinatorial inhibition of miR-1a and miR-15b drives cardiomyocyte proliferation in P1 and P7 rat cardiomyocytes, hence the inventors examined if the inhibition of miR-1a and miR-15b could similarly increase cardiomyocyte proliferation in response to MI. In order to determine whether LNA-1a and LNA-15b in adult mouse heart is able to enhance cardiomyocyte proliferation, the inventors examined Ki67 in cardiomyocytes in mouse heart after LNA-1a and LNA-15b or control LNA injection and MI. We detected increased Ki67 signal in cardiomyocytes after LNA-1a and LNA-15b injection in both control mice and MI mice compared to control LNA (Figure 6A and 6B). Furthermore, the inventors examined the expression of pHH3, and found a similar increased pHH3 signal in mouse hearts after LNA-1a/15b injection and MI (Figure 6C and 6D). In addition, the inventors examined the cytokinesis marker Aurora B, and found that LNA-1a/15b induced the expression of Aurora B in mouse cardiomyocytes in in control and MI (Figure 6E and 6F). Together, these data indicate that LNA-1a/15b could induce cardiomyocyte proliferation in response to MI in mouse *in vivo.*

### Example 5: Inhibition of miR-1a and miR-15b enhances cardiomyocyte proliferation in hiPSC-CMs

To test the combination in human cardiomyocytes, the inventors treated either the combination LNAs (LNA-1a, LNA-15b) or negative control LNA (LNA-control) in post-mitotic 40-day-old human induced pluripotent stem cell-derived cardiomyocytes (hiPSC-CMs). The inventors observed that the LNA-1a and LNA-15b increased cardiomyocyte proliferation under both normoxic and hypoxic conditions (Figure 7A and 7B). The miRNA expression levels were detected by quantitative real time PCR (Figure 7C and 7D).

### Example 6: Combination inhibition of miR-1a and miR-15b enhances cardiomyocyte proliferation and improves cardiac function in human mimetic tissues

Next, the inventors determined if combination of miR-1a and miR-15b affects cardiomyocyte proliferation and cardiac contractility in human monoculture cardiac organoids (Figure 8A). The inventors treated the combination of LNA-1a and LNA-15b or LNA-control and culture them in normoxia and hypoxia for 3 days in human cardiac organoids. Quantitative real time PCR showed that expression levels of miR-1 and miR-15b were significantly downregulated by LNAs treatment in monoculture cardiac organoids (Figure 8B and 8C). The cardiomyocyte proliferation was determined by EdU incorporation and pHH3. As shown in Figure 8D and 8E, the inventors found that the combination LNAs (miR-1a, miR-15b) increased cardiomyocyte proliferation in hypoxia based on increased EdU incorporation. Moreover, pHH3 showed that combination LNA-1a/15b treatment enhanced cardiomyocyte proliferation under hypoxic conditions, but not in the normoxia (Figure 8F and 8G). Furthermore, Hypoxia-reduced contractility was rescued by miR-1a and miR-15b inhibition (Figure 8H). Together, these data show that combination inhibition of miR-1a and miR-15b could increase cardiomyocyte proliferation and enhance contractility in human cardiac mimetics.

### Example 7: Combination inhibition of miR-1a, miR-27b and miR-34a induces cardiomyocyte proliferation

Furthermore, Dimmeler has shown that miR-34a inhibition is able to induce cardiomyocyte proliferation and improve cardiac function. Our data showed that miR-1a and miR-27b are critical for cell division in cardiomyocytes. Therefore, the inventors want to detect whether the combination inhibition of miR-1a, miR-27b and miR-34a is able to drive cardiomyocytes proliferation *in vitro* and *in vivo.* First, isolated neonatal rat cardiomyocytes were treated with combination of LNA-1a, LNA-27b and LNA-34a or LNA-control for 2 days, the cells were stained by Aurora B, α-actinin and DAPI. Combination of LNA-1a, LNA-27b and LNA-34a treatment remarkably increased cardiomyocytes proliferation in neonatal rat cardiomyocytes by significantly increased Aurora B-positive cardiomyocytes in telophase (Figure 9A and 9B). Then the inventors asked whether the combination inhibition of miR-1a, miR-27b and miR-34a could also promote cardiomyocytes proliferation, the 12-week-old mice were injected with either combination of LNA-1a, LNA-27b and LNA-34a or LNA-control for 1 week. As shown in the Figure 9C and 9D, combination of LNA-1a, LNA-27b and LNA-34a increased cardiomyocytes proliferation by increased Ki67-positive cardiomyocytes. Together, the data showed that combination of LNA-1a, LNA-27b and LNA-34a is able to drive cardiomyocytes proliferation *in vitro* and *in vivo.*

### Materials and Methods

### Animals

All animals were obtained from Janvier (Le Genest Saint-Isle, France). All animals used in this experiment were housed in a temprature-controlled room with a 12-h light-dark cycle and were allowed free access to food and water in agreement with NIH animal care guidelines, §8 German animal protection law, German animal welfare legislation and with the guidelines of the Society of Laboratory Animals (GV-SOLAS) and the Federation of Laboratory Animal Science Associations (FELASA).

### Myocardial infarction (MI)

MI was performed in 12-week-old male C57BL/6J mice, and was induced by permanent ligation of left anterior descending coronary artery (LAD). The mice were monitored up to 28 days after surgery, and the heart dimensions and cardiac function were determined by echocardiography on days 0, 7, 14, 21, and 28. 4 hours after MI, in vivo miRCURY LNA power inhibitors were injected intraperitoneally (i.p.) into mice at a dose 10 mg/kg. The following *in vivo* miRCURY LNA Power Inhibitors were purchased from QIAGEN: miR-1a-3p (339203 YCI0200659-FZA), miR-15b-5p (339203 YCI0200641-FZA), and negative control LNA (339203 YCI0200578-FZA).

### Isolation and maintenance of primary neonatal rat cardiomyocytes

Mated female Sprague Dawley rats were obtained from Janvier (Le Genest Saint-Isle, France). Primary neonatal rat cardiomyocytes (NRCs) were isolated from P1 and P7 rat pups by using the neonatal heart dissociation kit (Miltenyi Biotec) and the gentleMACS Dissociator according to the manufacturer's instruction. Isolated cells were pre-plated in plating medium (DMEM high glucose, M199 EBS (BioConcept), 10% horse serum, 5% fetal calf serum, 2% L-glutamine and 1% penicillin/streptomycin) for 1.5 h in 10 cm cell culture dishes (Nunc) at 37°C and 5% CO₂ at humidified atmosphere to get rid of fibroblasts as described previously [20]. NRCs were plated on Type-I bovine Collagen-coated (Advanced Biomatrix) plates or dishes in plating medium. 24 h after isolation of NRCs, the plating medium was changed to maintenance medium (DMEM high glucose, M199 EBS, 1% horse serum, 2% L-glutamine and 1% penicillin/streptomycin).

### In vitro administration of locked nucleic acids (LNA)

miRCURY LNA Power Inhibitors were directly added to the cell culture medium at a final concentration of 50 nM. All miRCURY LNA Power Inhibitors and negative control LNA were purchased from QIAGEN: miR-1a-3p (1999990-801), miR-15a-5p (339146 YCI0200640-FDA), miR-15b-5p (339146 YCI0200641-FDA), miR-16 (339146 YCI0200647-FDA), miR-27b-5p (339146 YCI0200648-FDA), miR-29a-3p (339146 YCI0200644-FDA), miR-34a-5p (339146 (YCI0200649-FDA), miR-34b-5p (339146 YCI0200645-FDA), miR-34c-5p (339146 YCI0200650-FDA), miR-92a-3p (339146 YCI0200646-FDA), miR-132-3p (1999990-801), miR-133a-3p (1999990-801), miR-155-3p (1999990-801), miR-195a-5p (339146 YCI0200642-FDA), miR-208a-3p (1999990-801), miR-497a-5p (339146 YCI0200643-FDA), mR-873a-5p (1999990-801), and negative control LNA CEL-CONTROL-INH (339147 YCI0199066-FFA).

### RNA isolation, reverse transcription and qRT-PCR

Samples were harvested in QIAzol Lysis Reagent (QIAGEN) and total miRNAs and total mRNAs were isolated with miRNA mini Kit (QIAGEN) according to the manufacturer's protocol. 200ng total RNAs were reverse transcript into cDNA using QuantiTect Reverse Transcription Kit (QIAGEN) according to the manufacturer's protocol. The Applied Biosystems StepOnePlus Real-Time PCR system (Applied Biosystems, CA, USA) with Fast SYBR∘,R Green Master Mix (Thermo Fisher Scientific) were used for analysis. TaqManTM Advanced miRNA Assays for mmu-miR-1a-3p (ID: mmu482914_mir), has-miR-1-3p (ID: mmu482914_mir), rno-miR-15a-5p (ID: rno483022_mir), mmu-miR-15b-5p (ID: mmu482957_mir), rno-miR-16-5p (ID: rno481312_mir), rno-miR-27b-5p (ID: rno481016_mir), hsa-miR-27b-sp (ID: 478789_mir), hsa-miR-29a-3p (ID: 478587_mir), hsa-miR-34a-5p (ID: rno481304_mir), hsa-miR_34c-5p(ID: 478052_mir), rno-miR-132-3p (ID: rno480919_mir), rno-miR-133a-3p (ID: rno481491_mir), mmu-miR-155-3p (ID: mmu481328_mir), mmu-miR-195a-5p (ID: mmu482953_mir), mmumiR-497a-5p (ID: mmu482607_mir), miR-873a-5p (ID: rno481425_mir), and hsa-miR-26a-5p (ID: 477995_mir).

### Preparation and maintenance of human iPSC-CMs

Human induced pluripotent stem cells (hiPSCs) were purchased from Cellular Dynamics International (CMC-100-010-001) and cultured as recommended by the manufacturer. The human iPSC-derived cardiomyocytes (hiPSC-CMs) were reprogrammed using the STEMdiff^{™} Cardiomyocyte Differentiation Kit (STEMCELL Technologies) according to the manufacture's protocol. Briefly, human iPS cells were plated at cell density of 3.5×10⁵ cells/well on Matrigel coated 12-well-plates using mTeSR^{™} medium supplemented with 5uM ROCK inhibitor (Y-27632, STEMCELL Technologies) for 24 h. After 1 day (-1), the medium was replaced with fresh TeSR^{™} medium. To induce cardiac differentiation, the TeSR^{™} medium is replaced with Medium A (STEMdiff^{™} Cardiomyocyte Differentiation Basal Medium containing Supplement A) at day 0, Medium B (STEMdiff^{™} Cardiomyocyte Differentiation Basal Medium containing Supplement B) at day 2, Medium C (STEMdiff^{™} Cardiomyocyte Differentiation Basal Medium containing Supplement C) at day 4 and day 6. On day 8, medium was switched to STEMdiff^{™} Cardiomyocyte Maintenance Medium with full medium changes every 2 days, to promote further differentiation into mature cardiomyocyte cells. All experiments were performed in the hiPSC-CMs at day 40. Hypoxic condition was achieved by using the Hypoxia chamber and the hiPSC-CMs were cultured at 3% O₂ for 2 days.

### Monoculture cardiac organoid formation Technique

Triculture organoids were created by hiPSC-CMs. Aggrewell^{™} 800 microwell culture plates were used to create the monoculture cardiac organoids in STEMdiff^{™} Cardiomyocyte Support Medium (STEMCELL Technologies). Approximately 900,000 wells in 2 ml cell suspension were pipetted into each well. After 2 days of culture, medium was switched to STEMdiff^{™} Cardiomyocyte Maintenance Medium for long term culture.

### Contractility measurement

Every single monoculture cardiac organoids was transferred into 96-well-plate and treated with either LNA-1a and LNA-15b or LNA-NC. Hypoxic condition was achieved by using the Hypoxia chamber and cardiac organoids were cultured at 1% O₂ for 3 days, then the contractility will be analyzed by lonOptix system. Units/pixels were determined by calibrating the system with a micrometer.

### Immunofluorescence staining

The NRCs were fixed with 4% paraformaldehyde (PFA)/PBS, the cells were permeabilized and incubated overnight at 4°C with primary antibodies against sarcomeric α-actinin (Sigma Aldrich), Aurora B (Abcam), or Ki67 (Abcam) diluted in 2% (v/v) HS/PBS. After 3 washes with PBS for 5 min, cells were incubated with 4',6-diamidino-2-phenylindole (DAPI; Thermo Fisher Scientific), AlexaFluor 555 anti-mouse (Thermo Fisher Scientific) and AlexaFluor 488 anti-rabbit (Thermo Fisher Scientific) secondary antibody for 1 h at room temperature. Dishes were mounted onto glass slides (Fisher Scientific) with a drop of ProLong Antifade (Thermo Fisher Scientific). Fluorescent images were acquired with the SP5 confocal microscopy (Leica) using a 40x magnification.

### Statistical Analysis

Data are represented as mean and error bars indicate the standard error of the mean (SEM). Two-tailed unpaired Student's t-tests (Excel) or one-way ANOVA analyses followed by either a Dunnett's multiple comparison post-test (multiple comparisons to a single control) or Bonferroni correction (multiple comparisons between different groups) were used as indicated in the respective figure legends. n.s. = not significant; *p < 0.05; **p < 0.01.

### Sequences of miRNA inhibitors

| LNAs | Sequence 5'-3' | SEQ ID NO |
|---|---|---|
| LNA-1a | ACTTCTTTACATTCC | 001 |
| LNA-15a | ACCATTATGTGCTGCT | 005 |
| LNA-15b | CCATGATGTGCTGCT | 002 |
| LNA-16 | TATTTACGTGCTGCT | 006 |
| LNA-27b | ACCAATCAGCTAAGCT | 003 |
| LNA-29a | GATTTCAGATGGTGCT | 007 |
| LNA-34a | AGCTAAGACACTGCC | 004 |
| LNA-34b | AGCTAATTACACTGCC | 008 |
| LNA-34c | AGCTAACTACACTGCC | 009 |
| LNA-92a | CGGGACAAGTGCAAT | 010 |
| LNA-132 | ATGGCTGTAGACTGTT | 011 |
| LNA-133a | GTTGAAGGGGACCAA | 012 |
| LNA-155 | ATGCTAACAGGTAGGA | 013 |
| LNA-195a | ATATTTCTGTGCTGCT | 014 |
| LNA-208a | CTTTTTGCTCGTCTTA | 015 |
| LNA-497a | ACCACAGTGTGCTGCT | 016 |
| LNA-873a | GACTCACAAGTTCCTG | 017 |
| Control LNA | TCCTAGAAAGAGTAGA | 018 |

## Claims

1. A combination of nucleic acid agents, wherein each of said nucleic acid agents is directed at and capable of specifically inhibiting and/or degrading a target microRNA, whereby the combination of nucleic acid agents is capable of inhibiting and/or degrading a combination of microRNAs within a cell, wherein said combination of microRNAs is
a. miR-1a, and
b. miR-15b, or
miR-15b and miR-27b;
for use in treatment or prevention of heart disease.

2. The combination of nucleic acid agents for use in treatment or prevention of heart disease according to claim 1, wherein said combination of microRNAs comprises miR-1a and miR-15b.

3. The combination of nucleic acid agents for use in treatment or prevention of heart disease according to any one of the preceding claims, wherein each nucleic acid agent comprises, or essentially consists of a hybridizing sequence of nucleotides, said hybridizing sequence of nucleotides being capable of forming a hybrid with a target microRNA.

4. The combination of nucleic acid agents for use in treatment or prevention of heart disease according to any one of the preceding claims, wherein each nucleic acid agent comprises, or essentially consists of, a sequence selected from the sequences SEQ ID NO 001-004.

5. The combination of nucleic acid agents for use in treatment or prevention of heart disease according to any one of the preceding claims, wherein one of said nucleic acid agents comprises or essentially consists of,
particularly wherein all of said nucleic acid agents comprise or essentially consist of, one or several locked nucleic acid (LNA) and/or peptide nucleic acid (PNA) moieties, particularly wherein said nucleic acid agents essentially consist of locked nucleic acid (LNA) moieties.

6. The combination of nucleic acid agents for use in treatment or prevention of heart disease according to any one of the preceding claims, wherein one of said nucleic acid agents comprises,
particularly wherein all of said nucleic acid agents comprise
one or several thiophosphate linkages.

7. The combination of nucleic acid agents for use in treatment or prevention of heart disease according to any one of the preceding claims, wherein one of said nucleic acid agents is,
particularly wherein all of said nucleic acid agents are,
expressed intracellularly from an exogenous (transgene) expression construct comprising a sequence encoding a hybridizing sequence of nucleotides under control of a promoter operable in a human myocardial cell.

8. An expression construct, or a plurality of expression constructs, encoding, under control of a promoter operable in a human myocardial cell, a combination of nucleic acid agents, wherein each of said nucleic acid agents is directed at and capable of specifically inhibiting and/or degrading a target microRNA, thereby inhibiting and/or degrading a combination of microRNAs, wherein said combination of microRNAs comprises
a. miR-1a, and
b. miR-15b, or
miR-15b and miR-27b;
for use in treatment or prevention of heart disease, particularly wherein said combination of microRNAs comprises miR-1a and miR-15b.

9. The combination of nucleic acid agents, or the expression construct, for use in treatment or prevention of heart disease according to any one of the preceding claims, wherein said nucleic acid agents or said expression construct are bound to a nanoparticle or encapsulated by a particulate structure selected from a virus and a liposome.

10. The combination of nucleic acid agents, or the expression construct, for use in treatment or prevention of heart disease according to claim 9, wherein said nanoparticle, or said particulate structure comprise a ligand facilitating delivery to or entry of the nanoparticle or particulate structure into a cardiomyocyte.

11. The expression construct, or the plurality of expression constructs, for use in treatment or prevention of heart disease according to any one of claims 8 to 10, wherein said promoter allows for heart-specific expression of said nucleic acid agents, particularly wherein said promoter is a heart-tissue-specific RNA Polymerase 2 promoter, particularly a promoter selected from Myosin Light Chain 2v (MLC2v) promoter, cardiac Troponin T (cTnT) promoter, Myosin Heavy Chain alpha (MHCa) promoter, and Titin (Ttn) promoter.

12. The combination of nucleic acid agents, or the plurality of expression constructs, for use in treatment or prevention of heart disease according to any one of the preceding claims, wherein said heart disease is **characterized by** a loss of cardiomyocytes, particularly said heart disease is heart failure **characterized by** a loss of cardiomyocytes, more particularly said heart failure is a result of myocardial infarction, stenosis, congenital disease, inflammation and/or sepsis.

13. The combination of nucleic acid agents, or the plurality of expression constructs, for use in treatment or prevention of heart disease according to any one of the preceding claims, wherein said heart disease is **characterized by** hypoxia of cardiomyocytes.

14. A pharmaceutical composition for use in treatment or prevention of heart disease comprising said combination of nucleic acid agents, or said plurality of expression constructs, according to any one of the preceding claims.

15. The pharmaceutical composition for use in treatment or prevention of heart disease according to claim 14, wherein the pharmaceutical composition is administered to the heart.

## Patentansprüche

1. Kombination von Nukleinsäurewirkstoffen, wobei jeder der Nukleinsäurewirkstoffe auf eine Ziel-microRNA gerichtet ist und in der Lage ist, diese spezifisch zu hemmen und/oder abzubauen, wodurch die Kombination von Nukleinsäurewirkstoffen in der Lage ist, eine Kombination von microRNAs innerhalb einer Zelle zu hemmen und/oder abzubauen, wobei die Kombination von microRNAs
a. miR-1a und
b. miR-15b oder
miR-15b und miR-27b ist;
zur Verwendung bei der Behandlung oder Vorbeugung von Herzerkrankung.

2. Kombination von Nukleinsäurewirkstoffen zur Verwendung bei der Behandlung oder Vorbeugung von Herzerkrankung gemäß Anspruch 1, wobei die Kombination von microRNAs miR-1a und miR-15b umfasst.

3. Kombination von Nukleinsäurewirkstoffen zur Verwendung bei der Behandlung oder Vorbeugung von Herzerkrankung gemäß einem der vorstehenden Ansprüche, wobei jeder Nukleinsäurewirkstoff eine hybridisierende Sequenz von Nukleotiden umfasst oder im Wesentlichen aus einer solchen besteht, wobei die hybridisierende Sequenz von Nukleotiden in der Lage ist, mit einer Ziel-microRNA einen Hybrid zu bilden.

4. Kombination von Nukleinsäurewirkstoffen zur Verwendung bei der Behandlung oder Vorbeugung von Herzerkrankung gemäß einem der vorstehenden Ansprüche, wobei jeder Nukleinsäurewirkstoff eine Sequenz umfasst oder im Wesentlichen aus einer solchen besteht, die aus den Sequenzen SEQ ID NO 001-004 ausgewählt ist.

5. Kombination von Nukleinsäurewirkstoffen zur Verwendung bei der Behandlung oder Vorbeugung von Herzerkrankung gemäß einem der vorstehenden Ansprüche, wobei einer der Nukleinsäurewirkstoffe umfasst oder im Wesentlichen besteht aus,
insbesondere wobei alle Nukleinsäurewirkstoffe umfassen oder im Wesentlichen bestehen aus einem oder mehreren Locked Nucleic Acid (LNA)- und/oder Peptidnukleinsäure (PNA)-Gruppen,
insbesondere wobei die Nukleinsäurewirkstoffe im Wesentlichen bestehen aus Locked Nucleic Acid (LNA)-Gruppen.

6. Kombination von Nukleinsäurewirkstoffen zur Verwendung bei der Behandlung oder Vorbeugung von Herzerkrankung gemäß einem der vorstehenden Ansprüche, wobei einer der Nukleinsäurewirkstoffe umfasst, insbesondere wobei alle Nukleinsäurewirkstoffe eine oder mehrere Thiophosphatbindungen umfassen.

7. Kombination von Nukleinsäurewirkstoffen zur Verwendung bei der Behandlung oder Vorbeugung von Herzerkrankung gemäß einem der vorstehenden Ansprüche, wobei einer der Nukleinsäurewirkstoffe, insbesondere wobei alle Nukleinsäurewirkstoffe intrazellulär von einem exogenen (transgenen) Expressionskonstrukt exprimiert wird/werden, das eine Sequenz umfasst, die eine hybridisierende Sequenz von Nukleotiden unter der Kontrolle eines in einer menschlichen Myokardzelle funktionsfähigen Promotors codiert.

8. Expressionskonstrukt oder eine Vielzahl von Expressionskonstrukten, das/die unter der Kontrolle eines in einer menschlichen Myokardzelle funktionsfähigen Promotors eine Kombination von Nukleinsäurewirkstoffen kodiert/kodieren, wobei jeder der Nukleinsäurewirkstoffe auf eine Ziel-microRNA gerichtet ist und in der Lage ist, diese spezifisch zu hemmen und/oder abzubauen, wodurch eine Kombination von microRNAs gehemmt und/oder abgebaut wird, wobei die Kombination von microRNAs
a. miR-1a und
b. miR-15b oder
miR-15b und miR-27b umfasst;
zur Verwendung bei der Behandlung oder Vorbeugung von Herzerkrankung, insbesondere wobei die Kombination von microRNAs miR-1a und miR-15b umfasst.

9. Kombination von Nukleinsäurewirkstoffen oder Expressionskonstrukt zur Verwendung bei der Behandlung oder Vorbeugung von Herzerkrankung gemäß einem der vorstehenden Ansprüche, wobei die Nukleinsäurewirkstoffe oder das Expressionskonstrukt an ein Nanopartikel gebunden sind oder von einer Partikelstruktur, ausgewählt aus einem Virus und einem Liposom, eingekapselt sind.

10. Kombination von Nukleinsäurewirkstoffen oder das Expressionskonstrukt zur Verwendung bei der Behandlung oder Vorbeugung von Herzerkrankung gemäß Anspruch 9, wobei der Nanopartikel oder die Partikelstruktur einen Liganden umfasst, der die Abgabe an oder Eindringen des Nanopartikels oder der Partikelstruktur in einen Kardiomyozyten erleichtert.

11. Expressionskonstrukt oder Vielzahl von Expressionskonstrukten zur Verwendung bei der Behandlung oder Vorbeugung von Herzerkrankung gemäß einem der Ansprüche 8 bis 10, wobei der Promotor eine herzspezifische Expression der Nukleinsäurewirkstoffe erlaubt, insbesondere wobei der Promotor ein herzgewebespezifischer RNA-Polymerase-2 Promotor ist,
insbesondere ein Promotor ausgewählt aus Myosin-Leichtketten 2v Promotor (MLC2v), kardialem Troponin-T Promotor (cTnT), Myosin-Schwerekette alpha-Promotor (MHCa) und Titin-Promotor (Ttn).

12. Kombination von Nukleinsäurewirkstoffen oder Vielzahl von Expressionskonstrukten zur Verwendung bei der Behandlung oder Vorbeugung von Herzerkrankung gemäß einem der vorstehenden Ansprüche, wobei die Herzerkrankung **gekennzeichnet ist durch** einen Verlust von Kardiomyozyten, insbesondere wobei die Herzerkrankung eine Herzinsuffizienz ist **gekennzeichnet durch** einen Verlust von Kardiomyozyten, insbesondere wobei die Herzinsuffizienz das Ergebnis eines Myokardinfarkts, einer Stenose, einer angeborenen Erkrankung, einer Entzündung und/oder Sepsis ist.

13. Kombination von Nukleinsäurewirkstoffen oder Vielzahl von Expressionskonstrukten zur Verwendung zur Behandlung oder Vorbeugung von Herzerkrankung gemäß einem der vorstehenden Ansprüche, wobei die Herzerkrankung durch Hypoxie der Kardiomyozyten gekennzeichnet ist.

14. Pharmazeutische Zusammensetzung zur Verwendung bei der Behandlung oder Vorbeugung von Herzerkrankung, umfassend die Kombination von Nukleinsäurewirkstoffen oder Vielzahl von Expressionskonstrukten gemäß einem der vorstehenden Ansprüche.

15. Pharmazeutische Zusammensetzung zur Verwendung bei der Behandlung oder Vorbeugung von Herzerkrankung gemäß Anspruch 14 wobei die pharmazeutische Zusammensetzung dem Herz verabreicht wird.

## Revendications

1. Combinaison d'agents acides nucléiques, dans laquelle chacun desdits agents acides nucléiques est dirigé vers et capable d'inhiber et/ou de dégrader spécifiquement un microARN cible, de sorte que la combinaison d'agents acides nucléiques est capable d'inhiber et/ou de dégrader une combinaison de microARN dans une cellule, dans laquelle ladite combinaison de microARN est
a. miR-1a, et
b. miR-15b, ou
miR-15b et miR-27b ;
pour une utilisation dans le traitement ou la prévention des maladies cardiaques.

2. La combinaison d'agents acides nucléiques pour une utilisation dans le traitement ou la prévention des maladies cardiaques selon la revendication 1, dans laquelle ladite combinaison de microARN comprend miR-1a et miR-15b.

3. La combinaison d'agents acides nucléiques pour une utilisation dans le traitement ou la prévention des maladies cardiaques selon l'une quelconque des revendications précédentes, dans laquelle chaque agent acide nucléique comprend ou consiste essentiellement en une séquence hybridante de nucléotides, ladite séquence hybridante de nucléotides étant capable de former un hybride avec un microARN cible.

4. La combinaison d'agents acides nucléiques pour une utilisation dans le traitement ou la prévention des maladies cardiaques selon l'une quelconque des revendications précédentes, dans laquelle chaque agent acide nucléique comprend ou consiste essentiellement en une séquence choisie parmi les sequences SEQ ID NO 001-004.

5. La combinaison d'agents acides nucléiques pour une utilisation dans le traitement ou la prévention des maladies cardiaques selon l'une quelconque des revendications précédentes, dans laquelle l'un desdits agents acides nucléiques comprend ou consiste essentiellement,
en particulier lorsque tous lesdits agents acides nucléiques comprennent ou consistent essentiellement,
d'un ou plusieurs fragments d'acide nucléique verrouillé (LNA) et/ou d'acide nucléique peptidique (PNA), en particulier lorsque lesdits agents acides nucléiques sont essentiellement constitués de fragments d'acide nucléique verrouillé (LNA).

6. La combinaison d'agents acides nucléiques pour une utilisation dans le traitement ou la prévention des maladies cardiaques selon l'une quelconque des revendications précédentes, dans laquelle l'un desdits agents nucléiques comprend, en particulier lorsque tous lesdits agents nucléiques comprennent une ou plusieurs liaisons thiophosphate.

7. La combinaison d'agents acides nucléiques pour une utilisation dans le traitement ou la prévention des maladies cardiaques selon l'une quelconque des revendications précédentes, dans laquelle l'un desdits agents acides nucléiques est, en particulier dans laquelle tous lesdits agents acides nucléiques sont, exprimé(s) de manière intracellulaire à partir d'une construction d'expression exogène (transgénique) comprenant une séquence codant pour une séquence d'hybridation de nucléotides sous le contrôle d'un promoteur fonctionnant dans une cellule myocardique humaine.

8. Construction d'expression, ou une pluralité de constructions d'expression, codant, sous le contrôle d'un promoteur fonctionnant dans une cellule myocardique humaine, une combinaison d'agents acides nucléiques, dans laquelle chacun desdits agents acides nucléiques est dirigé vers et capable d'inhiber et/ou dégrader un microARN cible, inhibant et/ou dégradant ainsi une combinaison de microARN, dans laquelle ladite combinaison de microARN comprend
a. miR-1a et
b. miR-15b, ou
miR-15b et miR-27b;
pour une utilisation dans le traitement ou la prévention des maladies cardiaques, en particulier lorsque ladite combinaison de microARN comprend miR-1a et miR-15b.

9. La combinaison d'agents acides nucléiques, ou la construction d'expression, pour une utilisation dans le traitement ou la prévention des maladies cardiaques selon l'une quelconque des revendications précédentes, dans laquelle lesdits agents acides nucléiques ou ladite construction d'expression sont liés à une nanoparticule ou encapsulés par une structure particulaire choisie parmi un virus et un liposome.

10. La combinaison d'agents acides nucléiques, ou la construction d'expression, pour une utilisation dans le traitement ou la prévention des maladies cardiaques selon la revendication 9, dans laquelle ladite nanoparticule ou ladite structure particulaire comprend un ligand facilitant l'administration ou l' entrée de la nanoparticule ou de la structure particulaire dans un cardiomyocyte.

11. La construction d'expression, ou la pluralité de constructions d'expression, pour une utilisation dans le traitement ou la prévention des maladies cardiaques selon l'une quelconque des revendications 8 à 10, dans laquelle ledit promoteur permet l'expression spécifique au coeur desdits agents acides nucléiques, en particulier lorsque ledit promoteur est un promoteur de l'ARN polymérase 2 spécifique au tissu cardiaque, en particulier un promoteur choisi parmi le promoteur de la chaîne légère de myosine 2v (MLC2v), le promoteur de la troponine T cardiaque (cTnT), le promoteur de la chaîne lourde de myosine alpha (MHCa) et le promoteur de la titine (Ttn).

12. La combinaison d'agents acides nucléiques, ou la pluralité de constructions d'expression, pour une utilisation dans le traitement ou la prévention des maladies cardiaques selon l'une quelconque des revendications précédentes, dans laquelle ladite maladie cardiaque est **caractérisée par** une perte de cardiomyocytes, en particulier ladite maladie cardiaque est une insuffisance cardiaque **caractérisée par** une perte de cardiomyocytes, plus particulièrement ladite insuffisance cardiaque est le résultat d'un infarctus du myocarde, d'une sténose, d'une maladie congénitale, d'une inflammation et/ou septicémie.

13. La combinaison d'agents acides nucléiques, ou la pluralité de constructions d'expression, pour une utilisation dans le traitement ou la prévention des maladies cardiaques selon l'une quelconque des revendications précédentes, dans laquelle ladite maladie cardiaque est **caractérisée par** une hypoxie des cardiomyocytes.

14. Composition pharmaceutique pour une utilisation dans le traitement ou la prévention des maladies cardiaques, comprenant ladite combinaison d'agents acides nucléiques ou ladite pluralité de constructions d'expression, selon l'une quelconque des revendications précédentes.

15. La composition pharmaceutique pour une utilisation dans le traitement ou la prévention des maladies cardiaques selon la revendication 14, dans laquelle la composition pharmaceutique est administrée au coeur.
